# EUROPEAN PATENT SPECIFICATION

(11) **EP 4 316 572 B1**
(45) Date of publication and mention of the grant of the patent: **29.04.2026**
(21) Application number: 22188182.4
(22) Date of filing: 01.08.2022
(51) Int. Cl.: A61N 1/39, A61N 1/04

(54) **DEFIBRILLATOR DEVICE**
DEFIBRILLATORVORRICHTUNG
DISPOSITIF DE DÉFIBRILLATEUR

(43) Date of publication of application: 07.02.2024
(73) Proprietor: Kiefer, Ida Maria, 8382 Hinnerup (DK)
(72) Inventor: Kiefer, Ida Maria, 8382 Hinnerup (DK); Kiefer, Martin, 8382 Hinnerup (DK)
(74) Representative: Larsen & Birkeholm A/S

(56) References cited:
- WO-A1-2018/232450
- WO-A1-2019/070516
- US-A1- 2011 046 688
- US-A1- 2014 222 095
- US-A1- 2018 169 426
- US-A1- 2018 207 435
- US-A1- 2019 044 362
- US-A1- 2019 117 989
- US-A1- 2021 093 877
- US-B2- 8 838 235

## Description

### Technical field of the invention

The present invention relates to a defibrillator device. In particular the present invention describes an Automated External Defibrillators (AED) designed to be charged by and used in conjunction with a mobile communication device (preferably, a personal mobile communication device).

### Background of the invention

Sudden cardiac arrest is one of the main causes of death. In Denmark alone, roughly 5.000 people die each year from sudden cardiac arrest. It is the main cause of death for individuals over the age of 55 and only 1/17 outside hospitals survives sudden cardiac arrest. The most effective treatment of sudden cardiac arrest is the use of cardiopulmonary resuscitation (CPR) together with defibrillation, like AED.

AEDs are portable devices designed to automatically check for life-threatening heart rhythms associated with sudden cardiac arrest and to send an electrical shock to the heart to try to restore a normal rhythm when shockable heart rhythms are detected. The two most common conditions treated by AEDs are Pulseless Ventricular tachycardia (aka VT or V-Tach) and Ventricular fibrillation (VF or V-Fib). AEDs are typically designed so that they can be used by a layman in situations where professional medical personnel are not available.

Given their potential to save lives, automated external defibrillators (AED) have been deployed and made available in a wide variety of public and private locations so that they are available in the event that a person in the vicinity goes into cardiac arrest. By way of example, AEDs may be found in corporate and government offices, shopping centers, airports, airplanes, restaurants, hotels, sports stadiums, schools, fitness centers and a variety of other locations where people may congregate. For instance, WO2019/070516 A1 discloses a defibrillator system for use in e.g. shopping centers, having a battery for storing sufficient energy to facilitate charging capacitor unit to level for delivering shock, and an interface unit operated in conjunction with defibrillator, and US 8 838 235 B2 discloses a wearable defibrillation system for heart arrhythmia patients with a communication module that is coupled to support structure and establishes local a communication link with a mobile communication device to exchange data.

Although the availability of AEDs has increased over the years, their relatively high cost tends to limit their distribution and there are still many locations including schools, sports fields, and a plethora of other places where people congregate don't have an on-site AED available. Furthermore, although many AEDs are considered "portable", most commercially available portable automated external defibrillators are bulky and heavy and they are rarely handled by people other than trained medical personnel.

Thus, there are many times, locations and events where no AED is available when a cardiac arrest incident occurs. Even when an AED is nearby when a sudden cardiac arrest incident occurs, the AED is often not used because either its presence is unknown, or the device seems intimidating to bystanders who are reluctant to try to use a device that they are unfamiliar with.

Although existing AEDs may save lives, there are continuing need to develop AEDs that are easy to use despite language, and that have characteristics likely to broaden the deployment and availability of automated external defibrillators.

### Summary of the invention

Furthermore, the AEDs current on the market, are traditionally configured to a select number of languages for voice instructions. The selected preferred language must be given to the manufacturer prior to operating the AED for configuration of the default used language. When operating the AED, the AED then gives the voice instructions in the preconfigured language, meaning if an AED in e.g., Sweden is used by a non-Swedish talking person, he or she will not be able to understand the given voice instructions. Utilizing a mobile device for voice instructions, the instructions given to the operator of the AED can then be localized using the default language of the mobile device. The operator of the AED then no longer depends on the language pre-selected by the manufacturer.

Accordingly, the present invention provides a defibrillator defined in claim 1, with further aspects and preferred embodiments being defined in the appended claims.

Thus, the present invention may relate to a defibrillator (as for example an automated external defibrillator) that can be powered by a mobile communication device such as a smart cellular phone or a tablet computer is described.

Utilizing a phone (or other mobile communication devices) as the power supply for an external defibrillator allows the external defibrillator to be smaller and, in this case, removes the need for a battery that stores sufficient energy for shock delivery-which would need to be checked and/or replaced on a regular basis.

Additionally, certain control functionality, computation, data processing, and user instructions are to be handled and presented by the mobile communications device thereby further simplifying the defibrillator design and improving the user experience.

This architecture takes advantage of compatible smart phones, tablets, computers and other mobile communication devices.

In an embodiment of the present invention, the defibrillator may be an AED suitable for use with an operator's personal smart phone and/or other types of personal portable communication or computing devices.

Yet another embodiment of the present invention, one or more of the features described herein may be well suited for use in more conventional defibrillator architectures that are not necessarily intended for use in conjunction with a mobile communication device by providing proper communication equipment into the traditional AED allowing proper communication between the AED and the personal mobile communication device.

In a further embodiment, the defibrillator may include a shock delivery capacitor and a charging circuitry. The charging circuitry may include a voltage boosting circuitry that boosts the voltage of received current to charge the shock delivery capacitor.

The defibrillator according to the present invention may comprise a charging circuitry. The charging circuitry may include current regulating circuitry configured to maintain a current draw from a power source for the voltage boosting circuitry throughout the charging of the capacitor. The current regulating circuitry may include a transitory electrical energy store that may serve as a temporary store for electrical energy drawn for a power source during the voltage boosting circuitry's current shut-off intervals and as a supply of supplemental current to the voltage boosting circuitry during at least portions of the periodic current draw intervals. In an embodiment, the current regulating circuitry may include a digitally controlled current limiting Buck converter.

The power source according to the present invention may be a mobile communication device, in particular a personal mobile communication device.

In an embodiment of the present invention, the current regulating circuitry may include a current sensor for sensing the current drawn from the power source and a controller (which may optionally be the defibrillation controller) that may receive a sensed input current from the current sensor and turns an input switch of the voltage boosting circuitry on and off to maintain the current drawn from the power source within a designated range throughout the charging of the capacitor.

Preferably, the defibrillator device may be arranged to automatically begin charging the capacitor when the defibrillator is initially activated. In an embodiment, the charging may automatically begin when the defibrillator may be initially connected to a mobile communication device. In yet an embodiment, the charging automatically may begin when the defibrillator may be manually activated by a user or in response to other specific triggers
In a further embodiment, the defibrillator may be connected to a mobile communication device through a connector cable that may be plugged into the mobile communication device. In another embodiment, the defibrillator and the mobile communication device may be connected wirelessly - as for example through the use of inductive charging and the use of a short-range wireless communication protocol.

In an embodiment of the present invention the defibrillator may include a bleed circuit that slowly drains the capacitor such that the capacitor may not retain a charge for a prolonged period of time. In an embodiment, the bleed circuit may be a voltage sensing circuit arranged to measure a voltage of the capacitor.

Various housings for the defibrillator device according to the present invention may be suitable. In an embodiment, the defibrillator may include a rectangular housing. The rectangular housing may have an external opening at a first end of the rectangular housing and one external opening at the second end of the rectangular housing. Defibrillator electronics may be positioned within the housing, e.g. the rectangular housing, and a removable end cap may be provided to cover the external opening. In an embodiment, a pair of defibrillator pads and/or an electrical connector cable may be stored with the housing and may be made accessible.

The end cap at each end may form a watertight seal with the first end of the rectangular housing. In an embodiment the end cap may have a pull feature configured to be pulled to remove the end cap from the ends of the housing.

In an embodiment, the defibrillator electronics may include a first circuit board that carries low voltage components and/or a second circuit board that carries high voltage electrical components.

The defibrilliator device according to the present invention may comprise a defibrillator discharge capacitor.

Various methods of charging the defibrillator discharge capacitor may be provided. In an embodiment, a maximum draw current for a discharge capacitor charging circuit may be set based at least in part on a current delivery capability of a connected power supply, such as a connected mobile communication device. In an embodiment, the defibrillator may be suitable for connection to multiple different types of mobile communication devices having different current delivery capabilities. In such embodiments, different maximum draw currents can be specified for charging the capacitor unit to facilitate efficient use of such devices.

In an embodiment, a maximum current through a primary coil of a transformer may set at the time of charging based at least in part on a current delivery capability of the power source. In a further embodiment, the maximum current through the primary coil may be changed during the charging of the defibrillator discharge capacitor based at least in part on a then present voltage or charge level of the defibrillator discharge capacitor.

In an embodiment, a variable electrical characteristic of a transitory electrical energy store may be changed during charging of the capacitor unit based on the discharge capacitor charge level. In yet an embodiment, an input switch of the voltage boosting circuit may be turned on and off to maintain the current drawn from the power source within a designated range.

In a further embodiment, charging of a shock delivery capacitor may be automatically initiated when the defibrillator unit is initially connected to the mobile communication device.

Another aspect of the present invention relates to one or more apps and/or other software or firmware-based control routines that may be well suited for controlling various aspects of the use and/or operation of a defibrillator. An app or other suitable software construct may have programmed instructions stored in the memory of a computing device such as a mobile communication device.

In an embodiment, the app provided on the mobile communication device may be configured to transmit an indication of a parameter to the defibrillator that may be indicative of, or can be used by the defibrillator to determine, the mobile communication device's current delivery capabilities. The app may include programmed instructions for analyzing heart rhythms received from the defibrillator unit to determine whether a patient has a shockable heart rhythm.

In another embodiment, the app may be configured to automatically authorize delivery of current from the computing device to the defibrillator unit in response to the connection of a defibrillator unit to the computing device.

In an embodiment, a defibrillator control app may be configured to generate an event history log that records a history associated with the use of an associated defibrillator for a particular event. The event history log may include a shock history that includes an indication of the number of shocks delivered, an indication of the energy charge utilized in each applied shock associated with the event and the time that each applied shock associated with the event was administered. The app may also be configured to display an event history GUI element on a display screen of the mobile communication device. Selection of the event history GUI element may cause an event history frame to be displayed on the display screen. The event history frame may show the number of shocks delivered, the energy charge utilized in each applied shock associated with the event and the time that each applied shock associated with the event was administered. The defibrillator control app may also be configured to send this information to a central informational database.

A preferred embodiment of the present invention relates to a defibrillator device capable of administering an electrical shock to a patient and operable via a personal mobile communication device, comprising the following;
the defibrillator device having a housing, the housing having external openings for connectors, wherein the defibrillator device further comprising connectors only for external components in forms of the mobile communication device and pads;
the housing having a compartment, reserved for the logic board that holds the electronic circuit and electronic components.

In an embodiment of the present invention the mobile communications device connector may provide power (for generating an electrical shock to a patient) from the mobile communication device, when coupled to the mobile communication device connector, to the defibrillator device.

In a further embodiment of the present invention the defibrillator device may not comprise an internal power supply and no internal or external power source in terms of a battery for generating an electrical shock to a patient.

In a further embodiment of the present invention the defibrillator may comprise defibrillator electronics, and the defibrillator electronics comprise the logic board for the high and low voltages with no connectors for internal or external batteries
In yet a further embodiment of the present invention the defibrillator may comprise one or more circuitry, and the circuitry includes one or more capacitors to store energy.

In another embodiment of the present invention the defibrillator may comprise defibrillator electronics, and the defibrillator electronics include a voltage circuit to charge a shock delivery capacitor with current received from the mobile device.

In a further embodiment of the present invention, the defibrillator further comprises one or more defibrillator pad connector, the one or more defibrillator pad connector being accessible through an external opening at an end of the housing.

In a further embodiment of the present invention the defibrillator may be configured to provide instructions for handling the defibrillator on the mobile communication device. Preferably the instructions may be visual instructions (like text, video, pictures, diagram or the like), audible instructions, or a combination hereof.

In an embodiment of the present invention the instructions may be given by the mobile device is in the default selected language of the mobile device.

In another embodiment of the present invention the defibrillator device may be operative with the addition of a mobile communication device and the audible instructions given by the mobile device is in the default selected language of the mobile device.

Preferably, all visual and/or audio user guidance may be executed on the mobile device.

A preferred embodiment of the present invention relates to the use of the defibrillator according to the present invention as an external defibrillator device.

### Brief description of the figure

The invention and the advantages thereof, may be further explained by reference to the following description taken in conjunction with the accompanying drawings in which:

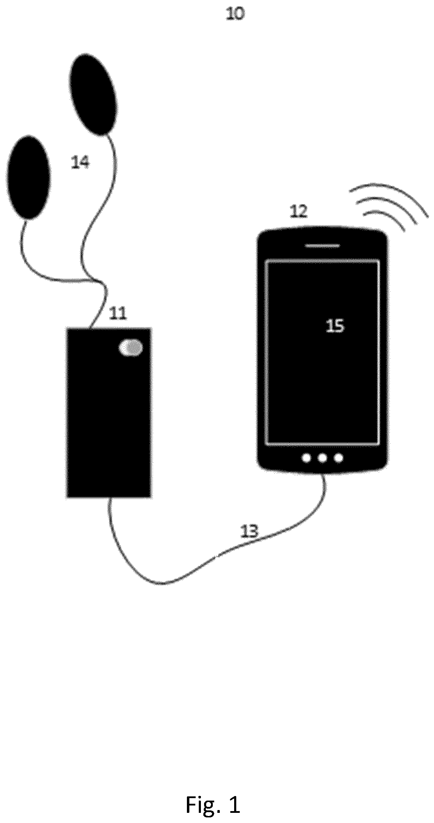

Fig. 1 shows a diagrammatic illustration of an automated external defibrillator system 10 ready for deployment in accordance with the present invention.

### Detailed description of the invention

Referring initially to fig. 1, a portable defibrillator architecture in accordance with an embodiment of the present invention is described. The illustrated architecture may be suited for use in automated external defibrillator and it may be suitable for use in manual defibrillators and in hybrid defibrillators that may be used in either automated or manual modes.

The core of the portable defibrillator system 10 is a defibrillation unit 11 which is used in conjunction with a mobile communication device 12 such as a cell phone, a tablet, a computer, a personal digital assistant (PDA) or other portable computing device. The system 10 also includes a connector cable 13 and pair of defibrillator pads 14. In the illustrated embodiment, the mobile communication device, may preferably be a personal mobile communication device, preferably selected from a smart phone, such as a Samsung Galaxy or an Apple iPhone. However, in another embodiment, a wide variety of other mobile communication devices may be used in place of the smart phone as long as the mobile communication device is using a supported operating system. Power for the defibrillation unit 11 may be obtained from the phone 12, which eliminates the need to provide and service batteries or other long term energy storage devices that store sufficient energy for shock delivery as part of the defibrillation unit.

The defibrillation unit 11 may be designed to be used in conjunction with an app 15 that may be installed, or installable on the mobile communication device. This permits use of the processing power of the phone to handle the signal processing, control and user interface functions required of the defibrillator system.

To use the defibrillator, the connector cable 13 may be plugged in to the I/O connector on the phone 12. The defibrillation unit 11 may be configured to begin charging the shock delivery circuitry as soon as it is plugged into the phone. In the illustrated embodiment, connector cable 13 takes the form of a USB cable. The cable can include any form that is appropriate for connection to the phone's I/O connector. A multiple number of cables, and/or adaptors, may be provided with the defibrillator device to ensure connection to various mobile communication devices.

Any of a variety of commercially available defibrillator pads may be used as defibrillator pads 14. Typically, the defibrillator pads may comprise an adhesive so that they can be securely attached to a patient at the time of a sudden cardiac event. If desired separate pads can be provided for adult and pediatric applications. The pads might be supplied in a sealed bag.

The medical community has established a variety of recommended external defibrillation shock protocols. These protocols typically call for the delivery of an electrical shock on the order of 120-200 joules at a voltage on the order of 1400-2000V for an adult when a biphasic defibrillator is used. More energy, as for example 200-360 joules is typically required if a monophasic defibrillator is used. Considerably lower shock intensities are recommended for pediatrics applications. The recommended shock guidelines can vary with the age/size of the patient and the nature of the heart rhythms that are detected. The defibrillator electronics can be configured to deliver any shock protocol deemed appropriate for the specific event.

It should be noted that embodiments and features described in the context of one of the aspects of the present invention also apply to the other aspects of the invention.

## Claims

1. A defibrillator device (11)
capable of administering an electrical shock to a patient and
operable via a personal mobile communication device (12), comprising the following;
a housing, the housing having external openings for connectors, wherein the defibrillator device further comprises connectors only for external components in forms of the mobile communication device and pads; the housing having a compartment, reserved for the logic board that holds the electronic circuit and electronic components and **characterized in that** the defibrillator device is configured to provide instructions given by the mobile communication device in the default selected language of the mobile communication device.

2. The defibrillator according to claim 1, wherein the mobile communications device connector provides power (for generating an electrical shock to a patient) from the mobile communication device, when coupled to the mobile communication device connector, to the defibrillator device.

3. The defibrillator according to anyone of claims 1-2, wherein the defibrillator device does not comprise an internal power supply and no internal or external power source in terms of a battery for generating an electrical shock to a patient.

4. A defibrillator as recited in anyone of claims 1-3 wherein the defibrillator comprises defibrillator electronics, and the defibrillator electronics comprise the logic board for the high and low voltages with no connectors for internal or external batteries

5. A defibrillator as recited in anyone of claims 1-4 wherein the defibrillator comprises one or more circuitry, and the circuitry includes one or more capacitors to store energy.

6. A defibrillator as recited in anyone of claims 1-5 wherein the defibrillator comprises defibrillator electronics, and the defibrillator electronics include a voltage circuit to charge a shock delivery capacitor with current received from the mobile device.

7. A defibrillator as recited in anyone of claims 1-6 further comprising one or more defibrillator pad connector, the one or more defibrillator pad connector being accessible through an external opening at an end of the housing.

8. The defibrillator according to claims 1-7 wherein defibrillator is configured to provide instructions for handling the defibrillator on the mobile communication device.

9. A defibrillator as recited in anyone of claims 1-8, wherein the defibrillator device being operative with the addition of a mobile communication device and the audible instructions given by the mobile device is in the default selected language of the mobile device.

## Patentansprüche

1. Eine Defibrillatorvorrichtung (11), die geeignet ist, einem Patienten einen elektrischen Schock zu verabreichen und über ein persönliches mobiles Kommunikationsgerät (12) betreibbar ist, umfassend Folgendes:
ein Gehäuse, wobei das Gehäuse externe Öffnungen für Steckverbinder aufweist, wobei die Defibrillatorvorrichtung ferner ausschließlich Steckverbinder für externe Komponenten in Form des mobilen Kommunikationsgeräts und von Pads umfasst;
wobei das Gehäuse ein Fach aufweist, das für die Logikplatine vorgesehen ist, welche die elektronische Schaltung und elektronische Komponenten trägt,
und **dadurch gekennzeichnet, dass** die Defibrillatorvorrichtung so konfiguriert ist, dass sie Anweisungen bereitstellt, die durch das mobile Kommunikationsgerät in der standardmäßig ausgewählten Sprache des mobilen Kommunikationsgeräts vorgegeben werden.

2. Der Defibrillator nach Anspruch 1, wobei der Steckverbinder für das mobile Kommunikationsgerät Leistung (zur Erzeugung eines elektrischen Schocks für einen Patienten) von dem mobilen Kommunikationsgerät bereitstellt, wenn er mit dem Steckverbinder des mobilen Kommunikationsgeräts gekoppelt ist, an die Defibrillatorvorrichtung.

3. Der Defibrillator nach einem der Ansprüche 1-2, wobei die Defibrillatorvorrichtung keine interne Stromversorgung und keine interne oder externe Stromquelle in Form einer Batterie zur Erzeugung eines elektrischen Schocks für einen Patienten umfasst.

4. Ein Defibrillator gemäß einem der Ansprüche 1-3, wobei der Defibrillator Defibrillatorelektronik umfasst und die Defibrillatorelektronik die Logikplatine für Hoch- und Niederspannungen ohne Steckverbinder für interne oder externe Batterien umfasst.

5. Ein Defibrillator gemäß einem der Ansprüche 1-4, wobei der Defibrillator eine oder mehrere Schaltungen umfasst und die Schaltung einen oder mehrere Kondensatoren zur Energiespeicherung einschließt.

6. Ein Defibrillator gemäß einem der Ansprüche 1-5, wobei der Defibrillator Defibrillatorelektronik umfasst und die Defibrillatorelektronik eine Spannungsschaltung umfasst, um einen Schockabgabekondensator mit vom mobilen Gerät empfangenem Strom aufzuladen.

7. Ein Defibrillator gemäß einem der Ansprüche 1-6, ferner umfassend einen oder mehrere Defibrillator-Pad-Steckverbinder, wobei der eine oder die mehreren Defibrillator-Pad-Steckverbinder durch eine externe Öffnung an einem Ende des Gehäuses zugänglich sind.

8. Der Defibrillator nach den Ansprüchen 1-7, wobei der Defibrillator so konfiguriert ist, dass er Anweisungen zur Handhabung des Defibrillators auf dem mobilen Kommunikationsgerät bereitstellt.

9. Ein Defibrillator gemäß einem der Ansprüche 1-8, wobei die Defibrillatorvorrichtung mit der Hinzufügung eines mobilen Kommunikationsgeräts betriebsfähig ist und die durch das mobile Gerät ausgegebenen akustischen Anweisungen in der standardmäßig ausgewählten Sprache des mobilen Geräts erfolgen.

## Revendications

1. Dispositif défibrillateur (11) apte à administrer un choc électrique à un patient et actionnable via un dispositif personnel de communication mobile (12), comprenant ce qui suit :
un boîtier, le boîtier comportant des ouvertures externes pour des connecteurs, le dispositif défibrillateur comprenant en outre uniquement des connecteurs pour des composants externes sous forme du dispositif de communication mobile et de pads ;
le boîtier comportant un compartiment réservé à la carte logique qui porte le circuit électronique et des composants électroniques,
et **caractérisé en ce que** le dispositif défibrillateur est configuré pour fournir des instructions données par le dispositif de communication mobile dans la langue sélectionnée par défaut du dispositif de communication mobile.

2. Le défibrillateur selon la revendication 1, dans lequel le connecteur du dispositif de communication mobile fournit une puissance (pour générer un choc électrique pour un patient) provenant du dispositif de communication mobile, lorsqu'il est couplé au connecteur du dispositif de communication mobile, au dispositif défibrillateur.

3. Le défibrillateur selon l'une quelconque des revendications 1-2, dans lequel le dispositif défibrillateur ne comprend pas d'alimentation électrique interne et aucune source d'alimentation interne ou externe sous forme de batterie pour générer un choc électrique pour un patient.

4. Un défibrillateur selon l'une quelconque des revendications 1-3, dans lequel le défibrillateur comprend une électronique de défibrillateur, et l'électronique de défibrillateur comprend la carte logique pour les hautes et basses tensions sans connecteurs pour des batteries internes ou externes.

5. Un défibrillateur selon l'une quelconque des revendications 1-4, dans lequel le défibrillateur comprend un ou plusieurs circuits, et le ou les circuits comprennent un ou plusieurs condensateurs pour stocker de l'énergie.

6. Un défibrillateur selon l'une quelconque des revendications 1-5, dans lequel le défibrillateur comprend une électronique de défibrillateur, et l'électronique de défibrillateur comprend un circuit de tension pour charger un condensateur de délivrance de choc avec un courant reçu du dispositif mobile.

7. Un défibrillateur selon l'une quelconque des revendications 1-6, comprenant en outre un ou plusieurs connecteurs de pads de défibrillateur, le ou les connecteurs de pads de défibrillateur étant accessibles à travers une ouverture externe à une extrémité du boîtier.

8. Le défibrillateur selon les revendications 1-7, dans lequel le défibrillateur est configuré pour fournir des instructions pour la manipulation du défibrillateur sur le dispositif de communication mobile.

9. Un défibrillateur selon l'une quelconque des revendications 1-8, dans lequel le dispositif défibrillateur est opératif avec l'ajout d'un dispositif de communication mobile et les instructions audibles données par le dispositif mobile sont dans la langue sélectionnée par défaut du dispositif mobile.
